## Europäisches Patentamt

(19) European Patent Office

Office européen des brevets

(11) Publication number: **0 129 434**
**B1**

(12) **EUROPEAN PATENT SPECIFICATION**

(45) Date of publication of patent specification: 06.09.89

(51) Int. Cl.⁴: **A 61 K 39/44,** A 61 K 39/395

(21) Application number: 84304096.5

(22) Date of filing: 18.06.84

(54) Immunotoxin conjugates.

<table>
<tr><td>

(30) Priority: 21.06.83 US 506540

(43) Date of publication of application:
27.12.84 Bulletin 84/52

(45) Publication of the grant of the patent:
06.09.89 Bulletin 89/36

(84) Designated Contracting States:
AT BE CH DE FR GB IT LI LU NL SE

(56) References cited:
EP-A-0 044 167
EP-A-0 055 115
EP-A-0 055 575
US-A-4 359 457

PROCEEDINGS OF THE NATIONAL ACADEMY
OF SCIENCES, vol. 80, October 1983, pages
6332-6335, US; E.S.VITETTA et al.: "Synergy of
ricin A chain-containing immunotoxins and
ricin B chain-containing immunotoxins in in
vitro killing of neoplastic human B cells"

</td><td>

(73) Proprietor: **BOARD OF REGENTS THE**
**UNIVERSITY OF TEXAS SYSTEM**
**201 West 7th Street**
**Austin Texas 78701 (US)**

(72) Inventor: **Uhr, Jonathan William**
**12355 Montego Plaza**
**Dallas Texas 75230 (US)**
Inventor: **Vitetta, Ellen S.**
**6914 Pemberton Drive**
**Dallas Texas 75230 (US)**

(74) Representative: **Bizley, Richard Edward et al**
**BOULT, WADE & TENNANT 27 Furnival Street**
**London EC4A 1PQ (GB)**

(56) References cited:
**NATURE, vol. 271, 23rd February 1978, pages**
**752-755, Macmillan Journals Ltd.; P.E.THORPE**
**et al.: "Toxicity of diphtheria toxin for**
**lymphoblastoid cells is increased by**
**conjugation to antilymphocytic globulin"**

</td></tr>
</table>

Courier Press, Leamington Spa, England.

# EP 0 129 434 B1

(58) References cited:

**NATURE, vol. 295, 18th February 1982, pages 604-605; K.A.KROLICK et al.: "Selective killing of leukaemia cells by antibody-toxin conjugates: implications for autologous bone marrow transplantation"**

**CHEMICAL ABSTRACTS, vol. 98, no. 9, 28th February 1983, page 54, no. 65458y, Columbus, Ohio, US; & US - A - 350 223 (UNITED STATES DEPT. OF HEALTH AND HUMAN SERVICES) 03-12-1982**

## Description

This invention relates to immunotoxin conjugates and their use to delete selectively a target population of cells. In particular, a toxin B chain moiety coupled to a cell surface affinity binding agent is useful in potentiating the cytotoxicity provided by a cell surface affinity binding agent coupled to a toxin A chain moiety.

Ricin is one of a number of plant proteins which, in minute quantities, exhibits considerable toxicity toward eukaryotic cells. Ricin is composed of two glycoprotein chains covalently linked via a single disulfide bond. The A chain of ricin, having an apparent molecular weight (AMW) of about 30,000, is responsible for the expression of toxicity, and acts enzymatically upon the 60S ribosomal subunit to produce irreversible abrogation of protein synthesis [Olsnes, et al., FEBS Lett 28, 48—50 (1972)]. Ricin B chain (AMW 32,000) functions as a lectin with specificity for galactose and serves to bind the toxin to the cell membrane [see, e.g., Baenziger, et al., J. Biol. Chem. 254, 9795—9799 (1979)].

The use of ricin, or the purified ricin A chain, in conjunction with antibodies, has been the subject of great interest as potentially-useful reagents in tumor therapy. Antibody-ricin and antibody-A chain conjugates, or "immunotoxins", have been used in a number of systems with varying degrees of success [see, e.g., Vitetta, et al., Science 219, 644—650 (1983); Thorpe, et al., Immunol. Rev. 62, 120—158 (1982); Neville, et al., Immunol. Rev. 62, 75—91 (1982); and Jansen, et al., Immunol. Rev. 62, 185—216 (1982)].

Procedures for deleting selected populations of cells by ricin A chain-antibody conjugates are well-recognized. The antibodies of choice are those which react with antigens on tumor cells or on subsets of normal lymphocytes. By deletion of the tumor cells, one may reduce, for example, tumor burdens in vivo [Krolick, et al,. J. Exp. Med. 155, 1797 (1982)] and remove tumor cells from bone marrow for autologous marrow transplantation [Thorpe, et al., Nature (London) 271, 752 (1978); and Krolick, et al., Nature (London) 295, 604 (1982)].

Also, by deleting normal subsets of lymphocytes. one may be able to "up" or "down" regulate the immune response. The advantage of immunotoxins is that they are highly specific for their target cell and that small doses can eliminate unwanted cells. Ricin A chain-antibody conjugates have been used primarily to delete normal and neoplastic B cells, both in vivo and in vitro. Certain laboratories have also used conjugates of ricin A chain and monoclonal antibody to eliminate neoplastic cells of T cell origin and a variety of other cancerous cells.

However, ricin A chain-antibody conjugates are not active when used against certain types of tumor cells (e.g., some T cell tumors) [Neville, et al., Immunol. Rev. 62, 75 (1982); and Thorpe, et al., Immunol. Rev. 62, 119 (1982)].

In contrast, immunotoxins coupled to the whole ricin toxin are much more potent cytocidal agents. Unfortunately, the presence of the galactose binding site of ricin B in intact ricin prevents its use in vivo because its target cell specificity thereby is lost. Attempts to overcome the nonspecificity of ricin-containing immunotoxins by blocking the galactose binding site are ongoing; however, their use in vivo has not been described yet.

Others have described studies in which ricin A chain-antibody conjugates can be potentiated by the addition of free B chain to cel cultures (Neville et al., supra). Researchers have postulated, therefore, that the B chain of ricin has two functions: (1) to facilitate entry of ricin into the cell by virtue of its galactose-binding properties, and (2) to allow the A chain to gain rapid access to the cytoplasm, perhaps by formation of a pore in the endocytic-vesicle membrane.

In a recent unpublished study, it has been discovered that injection of mice with nontoxic ricin A chain, followed 4—8 hours later by injection with non-toxic ricin B chain, produces ricin-induced death. This probably occurs by reformation of the intact ricin molecule in the serum or on the surface of circulating cells. It is believed, therefore, that the B chain plays an active role in potentiating the toxic activity of the ricin A chain.

In accordance with the invention, there are provided compositions and a method for potentiating the cytotoxic activity of cell surface binding agent-toxin conjugates while at the same time retaining target cell specificity. The compositions provided by the present invention include a selective binding agent coupled to a toxin B chain moiety.

Further, there is provided a composition comprising, in combination, a first conjugate including a selective binding agent coupled to a toxin B chain moiety together with a second conjugate including a cell surface binding agent coupled to a toxin A chain moiety. The selective binding agent of the first conjugate can be either a cell surface binding agent or a binding agent specific for the cell surface binding agent of the second conjugate.

In one aspect of the invention, there is provided a conjugate which encompasses an antibody as the cell surface binding agent coupled to a ricin B chain moiety. Further, there is provided a composition comprising a combination a first conjugate of an antibody coupled to a ricin B chain moiety together with a second conjugate of an antibody coupled to a ricin A chain moiety.

In yet another aspect of the invention there is provided a method for eliminating target cells from a population of cells containing such target cells by contacting the population of cells with a first conjugate comprising an affinity binding agent which is specific for an antigenic determinant on such target cells and which is coupled to a toxin B chain moiety and a second conjugate comprising a cell surface affinity binding agent specific to a different determinant on the same target cell and which is coupled to a toxin A chain moiety. The mixture of such conjugates potentiates the selective cytotoxic activity provided by the conjugate comprising a cell surface affinity binding agent coupled to toxin A chain moiety alone.

In addition, there is provided a process for preparing a toxin B chain conjugate which comprises covalently coupling a toxin B chain moiety to an antibody.

As a preferred embodiment, this invention provides a conjugate comprising an antibody coupled to toxin B chain moiety. Further, this invention provides a method for eliminaging target cells using, in concert, a composition comprising a first conjugate containing ricin B chain moiety and a second conjugate containing ricin A chain moiety.

Ricin is one of a number of toxin proteins which, in minute quantities, exhibits considerable toxicity toward cells. Ricin toxin is composed of two different glycoprotein chains covalently linked via a single disulfide bond. The A chain of ricin (AMW 30,000) is responsible for the toxicity caused by irreversible abrogation of protein synthesis. Ricin B chain (AMW 32,000) functions us a lectin which binds to galactose-containing glycoproteins or glycolipids exposed on cell surfaces. The general structure and mode of action exhibited by ricin is present in a variety of plant toxin proteins such as abrin, modeccin, pokeweed mitogen factor, and viscumin, and bacterial toxin proteins such as cholera, *E. coli*. heat-labile, pertussis, tetanus, botulinum, pseudomonas, shigella, and diphtheria toxins.

The ricin B conjugate and the ricin A conjugate used in the methods of this invention each comprise two active moieties: a cell surface or selective binding agent and a toxin A or B chain subunit covalently joined, preferably via a coupling agent. In each composition, one of the active moieties is represented by a molecule having binding affinity to a surface structure of a target cell or binding affinity to cell surface binding agent of the toxin A chain conjugate. Typically such molecules may be substances such as hormones, growth factors, lectins, or antibodies. The molecules of choice are antibodies or fragments thereof (in particular, Fab fragments) having cell surface binding affinity.

Monoclonal antibodies are preferred but not essential. Immunoglobulin fractions from serum can be used, albeit with a lesser degree of target specificity. Since the immunoglobulin fraction of an antiserum contains a multitude of antibodies directed to a wide range of divergent antigens, a practical usefulness of the compositions of this invention and the defined method for eliminating target cells dictates the need to isolate a desired collection of antibodies, each directed to a surface antigenic determinant present on the particular target cell.

An effective collection of such antibodies can be obtained by passing the immunoglobulin fraction over a column containing the respective antigen chemically coupled to a matrix. Antibody specific to the antigen will be retained on the column while unrelated immunoglobulin passes through. The retained antibody then can be collected by elution from the column using suitable eluting agents, such as acidic buffers of chaotropic agents. One should note that the isolated immunoglobulin, although directed to a single antigen, is not homogeneous. It comprises antibodies directed to a variety of antigenic determinants present on the antigen molecule. Consequently, the possibility exists for cross-reaction with other related antigens.

Therefore, use of monoclonal antibodies in preparing the compositions of this invention is highly preferred because they are directed to only one of possibly many antigenic determinants present on an antigen. Monoclonal antibodies are available by recognized methodology from hybridomas derived from lymphocytes present in the spleen or other organs.

Moreover, the use of monoclonal antibodies in the compositions used in the method of this invention carries the highly desirable feature of enhanced selectivity. Therefore, that both the ricin B conjugate and the ricin A conjugate be comprised of a monoclonal antibody is highly preferred. This ensures a high level of target cell specificity.

The preceding paragraphs have reference to one aspect of the present invention, the use of the same cell surface affinity binding agent for construction of both the ricin A chain conjugate and the ricin B chain conjugate. A greater degree of specificity, however, can be attained. Because a normal or tumor cell bears several different surface markers, one may be able to target the ricin A and the ricin B chains to such cells by coupling each to an antibody directed against a different determinant on the same cell. For example, in the case of a neoplastic B cell bearing both surface Ia (sIa) and surface Ig(sIg), immunotoxins against the sIa and the sIg idiotype can be prepared with the ricin B chain and ricin A chain, respectively. In the case of T cell tumors, a number of monoclonal antibodies exist which are reactive with subsets of human T cells. By using selected combinations of antibodies, one may target the ricin A and ricin B chains to specific subsets of such cells. Preferably, one antibody (coupled to ricin A chain) would define the subset, and the second (coupled to ricin B chain) would be a more general marker common to many subsets of cells. The B chain immunotoxin, directed against the more common marker, would bind also to normal cells; however, they would not be deleted. In contrast, the A chain immunotoxin would be focused only on the tumor cell and would be potentiated by B chain-containing immunotoxin.

Another approach contemplated by the present invention involves first directing a tumor cell reactive antibody-ricin A chain conjugate to tumor cells *in vivo*. The antibody preferably is univalent, *e.g.* F(ab')-A, and, therefore, is unable to cap and modulate. After the antibody-ricin A conjugate has been injected into a cancer-bearing patient and the excess eliminated from the recipient by excretion or degradation, a ricin B chain-containing immunotoxin directed against the antibody of the ricin A conjugate is injected. Only those cells which had bound the first immunotoxin would focus the second immunotoxin on the first. Therefore, such cells would be selectively deleted. The second immunotoxin preferably is a divalent anti-antibody, such as a F(ab')$_2$-B, which would not bind to macrophages, monocytes, or other cells bearing Fc receptors. Furthermore, since the B chain-containing immunotoxin would be innocuous if nonspecifically bound to a cell which had not previously bound the first immunotoxin, any side effects caused by the administration of the second immunotoxin would be eliminated. In contrast, cells binding both immunotoxins would be killed.

As noted, the ricin B-containing composition of this invention and the ricin A-containing composition used in the method of this invention each comprises at least two separate active moieties, one of which affords binding affinity (BA) and the other of which is a ricin subunit (RS), whether ricin A (RA) or ricin B (RB). These are joined through a coupling reagent, the requirements of the resulting composition being (a) the presence of at least one of each class of moiety, and (b) the retention of the innate activity of at least one of each class of moiety.

Other toxin proteins may be similarly coupled to the binding agent component for use in accordance with the present invention. Due to the similarity in their structure and mode of action, plant or bacterial toxin proteins such as abrin, modeccin, pokeweed mitogen factor, viscumin, and cholera, *E. coli*. heatlabile, pertussis, tetanus, botulinum, pseudomonas, shigella and diphtheria toxins may be utilized. Further, it may be advantageous to couple the A chain from abrin, for example, to a cell surface binding moiety to form the first conjugate of the invention and the B chain from viscumin, for example, to a selective binding agent moiety to form the second conjugate. It may be advantageous to use a plant protein toxin such as gelonin, which consists only of A chain, as the A chain to be coupled to the cell surface binding moiety to form the first conjugate. This first conjugate may be used then with a conjugate comprising a selective binding moiety coupled to a B chain selected from any one of the toxins ricin, viscumin, modeccin or abrin.

In accordance with these limitations, the compositions of this invention and those used in the method of this invention can be dimeric (BA-RS), *i.e.*, contain one of each class of moiety; trimeric [(BA$_2$-RS) or (BA-RS$_2$)], *i.e.*, contain two of one class of moiety and one of the other; tetrameric [(BA$_3$-RS), (BA$_2$RS$_2$), or (BA-RS$_3$)]; and the like.

As noted, highly preferred compositions for use in the method of this invention are those in which the binding moiety is antibody or an antigen binding fragment of antibody, and preferably a monoclonal antibody or an antigen binding fragment thereof. Typical compositions may be Ab-RB, Ab$_2$-RB, Ab-RB$_2$, Ab$_3$-RB, Ab$_2$-RB$_2$, Ab-RB$_3$, Ab-RA, Ab$_2$-RA, Ab-RA$_2$, Ab$_3$-RA, Ab-RA$_2$, or Ab-RA$_3$.

In preparing the compositions of this invention, the BA and RS moieties are joined via a suitable coupling reagent. A wide variety of coupling agents is reported in Ghose, T., and Blair, A. H., *J. Natl. Cancer Inst. 61*, 657—676 (1980). These authors report the use of carbodiimides as well as other bifunctional reagents, such as glutaraldehyde, *p*-benzoquinone, *p,p'* - difluoro - *m,m'* - dinitrodiphenylsulfone, or dimethyl adipimidate, for coupling antibody to cytotoxic agents. Because it is highly desirable to preclude formation of homopolymers, e.g., (BA)$_n$ or (RS)$_n$, use of a heterobifunctional reagent is preferred, ensuring formation of compositions having at least one of each class of moiety. Examples of such heterobifunctional reagents may be N - succinimidyl - 3 - (2 - pyridyldithio)propionate (SPDP), *m* - maleimidobenzoyl - N - hydroxy - succinimidyl ester, bromoacetyl - *p* - aminobenzoyl - N - hydroxy - succinimidyl ester, or iodoacetyl - N - hydroxysuccinimidyl ester.

For example, using SPDP as coupling agent, a process for preparing a composition of this invention comprises (a) separately modifying both Ab and RS by reaction with SPDP, (b) reducing the Ab-containing product, (c) causing formation of the composition by mixing the Ab-containing and RS-containing products, and (d) separating non-reacted monomers by gel filtration.

The conjugates of this invention containing ricin B, when used in concert with ricin A conjugates, have general applicability in the specific and selective killing of a cell type defined by particular antigenic markers. By appropriate selection of the antigenic marker the cell surface binding agent can be directed to either a set of normal cells or to a subset of neoplastic cells bearing a distinguishing determinant. As such, they are useful, for example, in the immunotherapy of cancer, for treating parasitic infections, and for treating a wide range of autoimmune diseases. Moreover, the compositions have several *in vitro* applications, including, for example, elimination of leukemic cells in bone marrow prior to autologous bone marrow transplantation; elimination of T cells in bone marrow prior to allogeneic bone marrow transplantation; and killing of wild types for selection of mutants.

The compositions of this invention can be used in a variety of pharmaceutical formulations and can be administered by a variety of conventional routes, such as intramuscular, intravenous, subcutaneous, and intraperitoneal. As used, the term "pharmaceutically-acceptable" means those agents useful in the chemotherapy of warm-blooded animals.

When administering the conjugate compositions parenterally or intraperitoneally, pharmaceutically-acceptable forms for injection may include sterile aqueous solutions or dispersions and sterile powders for reconstitution into sterile injectable solutions or dispersions. The carrier can be a solvent or dispersing medium containing, for example, water, ethanol, polyol (for example glycerol, propylene glycol, or liquid polyethylene glycol) suitable mixtures thereof, and vegetable oils. Proper fluidity can be maintained, for example, by the use of a coating such as lecithin, by the maintenance of the required particle size in the case of dispersions and by the use of surfactants. Various antibacterial and antifungal

EP 0 129 434 B1

agents, for example, parabens, chlorobutanol, phenol, sorbic acid, and the like may also be used. In many cases, including isotonic agents, for example, sugars, sodium chloride, and the like will be desirable. Prolonged absorption of the injectable pharmaceutical form can be brought about by the use of agents delaying absorption, for example, aluminum monostearate and gelatin.

Sterile injectable solutions can be prepared by associating the conjugate composition defined earlier in the required amount of the appropriate solvent with any other ingredient as desired.

If desired, for more effective distribution, the compositions can be incorporated into slow release systems such as polymer matrices, liposomes, and microspheres. Moreover, the compositions can be administered either alone or as a mixture of a plurality of active ingredients.

Doses of the compositions are administered to the recipient for a period during which a therapeutic response is desired. The weight of the recipient and mode of administration will determine the size of the dose necessary to induce the desired response.

Especially advantageous is to formulate the conjugate compositions in unit dosage form for ease of administration and uniformity of dosage. Unit dosage form refers to a physically discrete unit suited as unitary dosages for the subject to be treated. Each unit contains a predetermined quantity of the composition calculated to produce the desired therapeutic effect in association with the pharmaceutically-acceptable carrier. The specific unit dosage form is dependent upon (a) the unique charactersitics of the particular composition and (b) the particular therapeutic effect to be achieved.

The following non-limiting examples are provided to further illustrate the invention.

I. Preparation of immunotoxins
A. Ricin A and B chain
The A and B chain subunits of ricin were purchased from Xoma Corporation, San Francisco, California. Prior to use, the A and B chains were dialyzed extensively at 4°C against phosphate buffered saline (PBS), pH 7.2. The recovery of the A and B chains were 50% and 80%, respectively.

B. Antibody
The selective and cell surface binding agent of this embodiment is affinity purified rabbit antihuman immunoglobulin (RaHIg) made according to protocol described in the literature (see, e.g. Muirhead, et al., Blood, 42, 327 (1983); Vitetta, et al., Science, 219, 644 (1983)).

C. Conjugation
10 µl of 60 mM dithiothreitol (DTT) in PBS was added to each mg of the dialyzed A or B chain. The mixtures were incubated at 25°C for 60 minutes and the reduced chains were separated from the DTT by gel filtration at 25°C on a Sephadex® G-25 column (18×1.5 cm) in PBS, pH 7.2. Antibodies were coupled as described in Vitetta, et al., Immunol. Rev. 62:159—183 (1982), and Carlsson, et al., Biochem. J., 173, 723 (1978), which are herein incorporated by reference. In particular, the antibody in PBS is treated with SPDP, N - succinimydyl - 3 - (2 - pyridyl)dithio)propionate at a pH of about 7.0 to 7.5 at a temperature of about 20°C to about 25°C. The antibody derivative then is treated with dithiothreitol in buffer solution. The thiolated antibody may be purified by gel filtration, if desired. The activated coupled antibodies were mixed with the freshly reduced A or B chains at an antibody:A chain or antibody:B chain molar ratio of 5:1. The mixture was incubated for 15 minutes at 4°C with gentle shaking and then dialyzed overnight against PBS at 4°C. The immunotoxins were concentrated to 1 mg/ml by pervaporation, dialyzed for 2—16 hours at 4°C against PBS, and centrifuged to remove insoluble material. The separation of the immunotoxin from the majority of free A chain, B chains and antibody was performed by gel filtration at 25°C on a Sephacryl S-200 column equilibrated with PBS, pH 7.2. Material with an apparent molecular weight of greater than 200,000 was pooled. Reduced and alkylated bovine alpha globulin, fraction 4 (Sigma), was added to a final concentration of 1 mg/ml to the pooled samples. The samples were stored 16—20 hours at 4°C prior to affinity purification.

The RaHIg-A chain (RaHIg-A) and RaHIg-B chain (RaHIg-B) immunotoxins were purified by affinity chromatography on Sepharose-HIg. For purification of the A chain-containing immunotoxins, the columns were equilibrated and washed in PBS, pH 7.2 and for the purification of the B chain-containing immunotoxins, the columns were equilibrated and washed in PBS, pH 7.6 containing 0.1M galactose. Samples were applied to the column and the fall-through was discarded. The columns were washed extensively in PBS (or PBS-0.1M gal) followed by 0.85% NaCl. Immunotoxins were eluted batchwise at 37°C with 2—3 column volumes of 3.5M MgCl₂. The MgCl₂ was removed by dialysis and the samples were concentrated by pervaporation to approximately 200—300 µg/ml. Reduced and alkylated bovine alpha globulin was added to a final concentration of 1 mg/ml. Samples were sterilized by filtration [on a filter prewetted with PBS 2% fetal calf serum (FCS)], and then aliquoted into sterile vials and stored at −20°C. Samples stored in this manner were stable for up to 4—6 months.

The recovery of the immunotoxin following affinity purification was 26—45% and each immunotoxin contained one or two A (or B) chain subunits per molecule of antibody. When immunotoxins were analyzed on SDS-PAGE slab gels using a sensitive silver staining technique, no free A or B chain in any of the immunotoxins was detected.

6

II. Use in ricin B chain containing conjugates and ricin A chain conjugates in the killing of neoplastic human B cells A. Neoplastic cell culture

The human Burkitt's lymphoma cell line, Daudi, as described in Houston, L. L., *Biochem. Biophys. Res. Commun.* 92:319—326 (1980), was maintained in suspension culture in RPMI-1640 supplemented with 10% (v/v) fetal calf serum (FCS), 20 mM fresh glutamine, and antibiotics. Cultures were maintained at 37°C in a humid incubator with a 95% $O_2$/5% $CO_2$ atmosphere. All cultures were used two days following subculture.

B. Treatment of Daudi cells

Two days after subculture, Daudi cells were harvested and washed in buffered saline solution (BSS). $10^5$ cells in BSS were distributed into microtiter wells. Dilutions of each of the two immunotoxins, RαHIg-A and RαHIg-B, or combination of both, were added to triplicate wells for 15 minutes at 4°C. Cells were centrifuged and washed three times in BSS. 200 μl or RPMI lacking leucine and containing 10% FCS were added to each well and the cells were resuspended by gentle agitation. Plates were cultured for 22 hours at 37°C in a 5% $CO_2$ incubator. Cells were then pulsed for 6 hours at 37° in 5% $CO_2$ with 5 μCi per well of $^3$H-leucine (New England Nuclear). Cells were harvested onto glass fiber discs and counted in a liquid scintillation spectrometer. Cells were treated with each of the immunotoxins individually as well as the following combinations of immunotoxins:

1. A nontoxic amount of RαHIg-A (0.3 μg/mg) plus different amounts of RαHIg-B, or
2. A nontoxic amount of RαHIg-B (0.5 μg/ml) followed by different amounts of RαHIg-A.

The results are tabulated in Table 1.

TABLE 1
% of cells remaining after treatment vs. control

| Conjugate(s) used | Concentration (μg/ml) | | | | | |
|---|---|---|---|---|---|---|
| | 0.03 | 0.05 | 0.3 | 0.5 | 1.3 | 2.6 |
| ITB* | 83 | 90 | 100 | 100 | 90 | 82 |
| ITA** | 100 | 90 | 82 | — | 22 | 18 |
| ITA+ITB (0.5 μg/ml) | 75 | 40 | 12 | — | 6 | 5 |
| ITB+ITA (0.3 μg/ml) | 95 | 64 | 50 | — | 20 | 10 |

*ITB=immunotoxin B conjugate (RαHIg-B)
**ITA=immunotoxin A conjugate (RαHIg-A)

As indicated in the Table, when Daudi cells were treated with 0.3 μg of RαHIg-A chain/$10^5$ cells, little toxicity was observed. No concentration of RαHIg-B was toxic. However, when 0.3 μg of the RαHIg-A was mixed with various combinations of RαHIg-B, there was significant cytotoxicity. It should be noted that this treatment of the Daudi cells with the mixture of immunotoxins was performed in BSS lacking galactose. As shown in Table 1, when Daudi·cells were treated with 0.5 μg of RαHIg-B, no toxicity was observed. In contrast, the RαHIg-A killed the Daudi cells in a dose-related manner. However, treatment of cells with 0.5 μg of RαHIg-B mixed with RαHIg-A was toxic to the cells even at those concentrations at which RαHIg-A itself was not toxic.

Although the conjugate compositions and methods have been described in terms of preferred embodiments, those skilled in the art will recognize that various changes may be made without departing from the intended scope of the invention.

**Claims for the Contracting States: BE, CH, DE, FR, GB, IT, LI, LU, NL, SE**

1. A conjugate comprising an antibody covalently coupled to a toxin B chain moiety.
2. A conjugate as claimed in Claim 1 in which the antibody is specific for a cell surface antigen.
3. A conjugate as claimed in Claim 1 or 2 in which the antibody is directed to a cell surface antigen of a tumor cell.
4. A conjugate as claimed in Claim 1 in which the antibody is directed against a second antibody.
5. A conjugate as claimed in any one of Claims 1 to 4 in which the toxin B chain is selected from ricin B chain, modeccin B chain, abrin B chain, pokeweed mitogen factor B chain, viscumin B chain, cholera toxin B chain, *E. coli* heat-labile toxin B chain, pertussis toxin B chain, botulinum toxin B chain, Pseudomonas toxin B chain, shigella toxin B chain or diphtheria toxin B chain.
6. A conjugate as claimed in Claim 5 in which the toxic B chain is ricin B chain.

7. A cytotoxic composition which comprises a first conjugate as claimed in any one of Claims 1 to 6, together with a second conjugate comprising an antibody covalently coupled to a toxin A chain moiety.

8. A composition as claimed in Claim 7 in which the second conjugate antibody is directed against a cell surface antigenic determinant.

9. A composition as claimed in Claim 7 or 8 in which each of the first and second conjugates comprises an antibody having identical specificity to a cell surface antigenic determinant.

10. A composition as claimed in Claim 7 or 8 in which the first conjugate antibody is directed against a cell surface antigenic determinant different from the cell surface antigenic determinant to which the second conjugate antibody is directed.

11. A composition as claimed in any one of Claims 7 to 10 in which each antibody is specific for a tumor cell antigenic determinant.

12. A composition as claimed in Claim 7 in which the second conjugate comprises an antibody specific for a cells surface antigenic determinant and the first conjugate comprises an antibody specific for the antibody of the second conjugate.

13. A composition as claimed in any one of Claims 7 to 12 in which the toxin A chain is selected from the A chain moiety of ricin, abrin, modeccin, gelonin, pokeweed mitogen factor, viscumin, cholera toxin, *E. coli* heat-labile toxin, pertussis toxin, botulinum toxin, Pseudomonas toxin, shigella toxin and diphtheria toxin.

14. A composition as claimed in Claim 13 in which the toxin A chain moiety is ricin A chain and the toxin B chain moiety is ricin B chain.

15. A pharmaceutical formulation which comprises a B chain conjugate as claimed in any one of Claims 1 to 6, associated with one or more pharmaceutically-acceptable carriers or vehicles therefor.

16. A product containing an A chain conjugate as defined in any one of Claims 7 to 14 and a B chain conjugate as claimed in any one of Claims 1 to 6 as a combined preparation for simultaneous, separate or sequential use in therapy.

17. An A chain conjugate as defined in any one of Claims 7 to 14 combined simultaneously, separately or sequentially with a B chain conjugate as claimed in any one of Claims 1 to 6 for use in therapy.

18. A process for preparing a toxin B chain conjugate as claimed in any one of Claims 1 to 6 which comprises covalently coupling a toxin B chain moiety to an antibody.

**Claims for the Contracting State: AT**

1. A process for preparing a toxin B chain conjugate which comprises covalently coupling an antibody to a toxin B chain moiety.

2. A process for preparing a conjugate as claimed in Claim 1 in which the antibody is specific for a cell surface antigen.

3. A process for preparing a conjugate as claimed in Claim 1 or 2 in which the antibody is directed to a cell surface antigen of a tumor cell.

4. A process for preparing a conjugate as claimed in Claim 1 in which the antibody is directed against a second antibody.

5. A process for preparing a conjugate as claimed in any one of Claims 1 to 4 in which the toxin B chain is selected from ricin B chain, modeccin B chain, abrin B chain, pokeweed mitogen factor B chain, viscumin B chain, cholera toxin B chain, *E. coli* heat-labile toxin B chain, pertussis toxin B chain, botulinum toxin B chain, Pseudomonas toxin B chain, shigella toxin B chain or diphtheria toxin B chain.

6. A process for preparing a conjugate as claimed in Claim 5 in which the toxin B chain is ricin B chain.

7. A process for preparing a cytotoxic composition which comprises associating a first conjugate as claimed in any one of Claims 1 to 6, together with a second conjugate comprising an antibody covalently coupled to a toxin A chain moiety.

8. A process for preparing a composition as claimed in Claim 7 in which the second conjugate antibody is directed against a cell surface antigenic determinant.

9. A process for preparing a composition as claimed in Claim 7 or 8 in which each of the first and second conjugates comprises an antibody having identical specificity to a cell surface antigenic determinant.

10. A process for preparing a composition as claimed in Claim 7 or 8 in which the first conjugate antibody is directed against a cell surface antigenic determinant different from the cell surface antigenic determinant to which the second conjugate antibody is directed.

11. A process for preparing a composition as claimed in any one of Claims 7 to 10 in which each antibody is specific for a tumor cell antigenic determinant.

12. A process for preparing a composition as claimed in Claim 7 in which the second conjugate comprises an antibody specific for a cell surface antigenic determinant and the first conjugate comprises an antibody specific for the antibody of the second conjugate.

13. A process for preparing a composition as claimed in any one of Claims 7 to 12 in which the toxin A chain is selected from the A chain moiety of ricin, abrin, modeccin, gelonin, pokeweed mitogen factor, viscumin, cholera toxin, *E. coli* heat-labile toxin, pertussis toxin, botulinum toxin, Pseudomonas toxin, shigella toxin and diphtheria toxin.

8

14. A process for preparing a composition as claimed in Claim 13 in which the toxin A chain moiety is ricin A chain and the toxin B chain moiety is ricin B chain.

15. A toxin B chain conjugate whenever prepared by a process according to any one of Claims 1 to 6.

**Patentansprüche für die Vertragsstaate: BE, CH, FR, GB, IT, LI, LU, NL, SE**

1. Konjugat, das einen Antikörper umfasst, der kovalent mit einem Toxin B-Kettenteil verknüpft ist.

2. Konjugat nach Anspruch 1, in dem der Antikörper spezifisch für ein Zelloberflächen-Antigen ist.

3. Konjugat nach Anspruch 1 oder 2, in dem der Antikörper auf ein Zelloberflächen-Antigen einer Tumorzelle gerichtet ist.

4. Konjugat nach Anspruch 1, in dem der Antikörper gegen einen zweiten Antikörper gerichtet ist.

5. Konjugat nach einem der Ansprüche 1 bis 4, in dem die Toxin B-Kette aus Ricin B-Kette, Modeccin B-Kette, Abrin B-Kette, Kermesbeerenmitogen - Faktor B-Kette, Viscumin B-Kette, Choleratoxin B-Kette, hitzelabile E. coli-Toxin B-Kette, Pertussistoxin B-Kette, Botulinumtoxin B-Kette, Pseudomonastoxin B-Kette, Shigellatoxin B-Kette oder Diphtheriatoxin B-Kette ausgewählt ist.

6. Konjugat nach Anspruch 5, in dem die Toxin B-Kette eine Ricin B-Kette ist.

7. Cytotoxische Zusammensetzung, die ein erstes Konjugat nach einem der Ansprüche 1 bis 6 zusammen mit einem zweiten Konjugat, das einen Antikörper umfasst, der kovalent an einen Toxin A-Kettenteil gebunden ist, umfasst.

8. Zusammensetzung nach Anspruch 7, in dem der zweite Konjugat-Antikörper gegen eine zelloberflächenantigene Determinante gerichtet ist.

9. Zusammensetzung nach Anspruch 7 oder 8, in der jedes der ersten und zweiten Konjugate einen Antikörper mit einer identischen Spezifität gegenüber einer zelloberflächenantigenen Determinante umfasst.

10. Zusammensetzung nach Anspruch 7 oder 8, in der der erste Konjugat-Antikörper gegen eine zelloberflächenantigene Determinante, die unterschiedlich gegenüber der zelloberflächenantigenen Determinante ist, auf die der zweite Konjugat-Antikörper gerichtet ist, gerichtet ist.

11. Zusammensetzung nach einem der Ansprüche 7 bis 10, in der jeder Antikörper spezifisch für eine tumorzellenantigene Determinante ist.

12. Zusammensetzung nach Anspruch 7, in der das zweite Konjugat einen spezifischen Antikörper für eine zelloberflächenantigene Determinante umfasst, und das erste Konjugat einen spezifischen Antikörper für den Antikörper des zweiten Konjugates umfasst.

13. Zusammensetzung nach einem der Ansprüche 7 bis 12, in der die Toxin A-Kette aus dem A-Kettenteil von Ricin, Abrin, Modeccin, Gelonin, Kermesbeerenmitogen-Faktor, Viscumin, Choleratoxin, hitzelabiles E. coli-Toxin, Pertussistoxin, Botulinumtoxin, Pseudomonastoxin, Shigellatoxin und Diphtheriatoxin ausgewählt ist.

14. Zusammensetzung nach Anspruch 13, in der der Toxin A-Kettenteil eine Ricin A-Kette ist und der Toxin B-Kettenteil eine Ricin B-Kette ist.

15. Pharmazeutische Formulierung, die ein B-Kettenkonjugat nach einem der Ansprüche 1 bis 6, verbunden mit einem oder mehreren pharmazeutisch verträglichen Trägern dafür umfasst.

16. Produkt, das ein A-Kettenkonjugat nach einem der Ansprüche 7 bis 14 und ein B-Kettenkonjugat nach einem der Ansprüche 1 bis 6 als ein kombiniertes Präparat für eine gleichzeitige, getrennte oder Folgeanwendung in der Therapie enthält.

17. A-Kettenkonjugat nach einem der Ansprüche 7 bis 14, das gleichzeitig, getrennt oder aufeinanderfolgend mit einem B-Konjugat nach einem der Ansprüche 1 bis 6 für die Verwendung in der Therapie kombiniert ist.

18. Verfahren zur Herstellung eines Toxin B-Kettenkonjugates nach einem der Ansprüche 1 bis 6, das das kovalente Kuppeln eines Toxin B-Kettenteils mit einem Antikörper umfasst.

**Patentansprüche für: AT**

1. Verfahren zur Herstellung eines Toxin B-Kettenkonjugates, das die kovalente Kupplung eines Antikörpers an einen Toxin B-Kettenteil umfasst.

2. Verfahren zur Herstellung eines Konjugates nach Anspruch 1, in dem der Antikörper spezifisch für ein Zelloberflächen-Antigen ist.

3. Verfahren zur Herstellung eines Konjugates nach Anspruch 1 oder 2, in dem der Antikörper auf ein Zelloberflächen-Antigen einer Tumorzelle gerichtet ist.

4. Verfahren zur Herstellung eines Konjugates nach Anspruch 1, in dem der Antikörper gegen einen zweiten Antikörper gerichtet ist.

5. Verfahren zur Herstellung eines Konjugates nach einem der Ansprüche 1 bis 4, in dem die Toxin B-Kette aus Ricin B-Kette, Modeccin B-Kette, Abrin B-Kette, Kermesbeerenmitogen-Faktor B-Kette, Viscumin B-Kette, Choleratoxin B-Kette, hitzelabile E. coli-Toxin B-Kette, Pertussistoxin B-Kette, Botulinumtoxin B-Kette, Pseudomonastoxin B-Kette, Shigellatoxin B-Kette oder Dipheriatoxin B-Kette gerichtet ist.

6. Verfahren zur Herstellung eines Konjugates nach Anspruch 5, in dem die Toxin B-Kette eine Ricin B-Kette ist.

7. Verfahren zur Herstellung einer cytotoxischen Zusammensetzung, das das Verbinden eines ersten Konjugates nach Anspruch 1 bis 6 mit einem zweiten Konjugat, das einen an einen Toxin A-Kettenteil kovalent gebundenen Antikörper umfasst, umfasst.

8. Verfahren zur Herstellung einer Zusammensetzung nach Anspruch 7, in dem der zweite Konjugat-Antikörper gegen eine zelloberflächenantigene Determinante gerichtet ist.

9. Verfahren zur Herstellung einer Zusammensetzung nach Anspruch 7 oder 8, in dem jedes der ersten und zweiten Konjugate einen Antikörper mit einer identischen Spezifität gegenüber einer zelloberflächenantigenen Determinante umfasst.

10. Verfahren zur Herstellung einer Zusammensetzung nach Anspruch 7 oder 8, indem der erste Konjugat-Antikörper gegen eine zelloberflächenantigene Determinante gerichtet ist, die von der zelloberflächenantigenen Determinante, auf die der zweite Konjugat-Antikörper gerichtet ist, verschieden ist.

11. Verfahren zur Herstellung einer Zusammensetzung nach einem der Ansprüche 7 bis 10, in dem jeder Antikörper spezifisch gegenüber einer tumorzellenantigenen Determinante ist.

12. Verfahren zur Herstellung einer Zusammensetzung nach Anspruch 7, in dem das zweite Konjugat einen für eine zelloberflächenantigene Determinante spezifischen Antikörper umfasst, und das erste Konjugat einen für den Antikörper des zweiten Konjugates spezifischen Antikörper umfasst.

13. Verfahren zur Herstellung einer Zusammensetzung nach einem der Ansprüche 7 bis 12, in dem die Toxin A-Kette aus dem A-Kettenteil von Ricin, Abrin, Modeccin, Gelonin, Kermesbeerenmitogen-Faktor, Viscumin, Choleratoxin, hitzelabiles E. coli-Toxin, Pertussistoxin, Botulinumtoxin, Pseudomonastoxin, Shigellatoxin und Diphtheriatoxin ausgewählt wird.

14. Verfahren zur Herstellung einer Zusammensetzung nach Ansrpuch 13, in dem der Toxin A-Kettenteil eine Ricin A-Kette und der toxin B-Kettenteil eine Ricin B-Kette ist.

15. Toxin B-Kettenkonjugat, hergestellt durch ein Verfahren gemäss einem der Ansprüche 1 bis 6.

**Revendications pour les états Contractants: BE, CH, FR, GB, IT, LI, LU, NL, SE**

1. Conjugué, comprenant un anticorps couplé par liaison covalente à un fragment chaîne B d'une toxine.

2. Conjugué selon la revendication 1, dans lequel l'anticorps est spécifique d'un antigène de surface cellulaire.

3. Conjugué selon la revendication 1 ou 2, dans lequel l'anticorps est dirigé contre un antigène de surface cellulaire d'une cellule tumorale.

4. Conjugué selon la revendication 1, dans lequel l'anticorps est dirigé contre un deuxième anticorps.

5. Conjugué selon l'une quelconque des revendications 1 à 4, dans lequel la chaîne B d'une toxine est choisie parmi la chaîne B de la ricine, la chaîne B de la modeccine, la chaîne B de l'abrine, la chaîne B de la lectine "pokeweed mitogen", la chaîne B de la viscumine, la chaîne B de la toxine du choléra, la chaîne B de la toxin thermolabile de *E. coli*, la chaîne B de la toxine de la coqueluche, la chaîne B de la toxine botulique, la chaîne B de la toxine de Pseudomonas, la chaîne B de la toxine de Shigella ou la chaîne B de la toxine de la diphtérie.

6. Conjugué selon la revendication 5, dans lequel la chaîne B d'une toxine est la chaîne B de la ricine.

7. Composition cytotoxique comprenant un premier conjugué selon l'une quelconque des revendications 1 à 6, avec un deuxième conjugué comprenant un anticorps couplé par liaison covalente à un fragment chaîne A d'une toxine.

8. Composition selon la revendication 7, dans laquelle le deuxième anticorps conjugué est dirigé contre un déterminant antigènique de surface cellulaire.

9. Composition selon les revendications 7 ou 8, dans laquelle chacun du premier et du deuxième conjugués comprend un anticorps ayant une spécificité identique à un déterminant antigénique de surface cellulaire.

10. Composition selon la revendication 7 ou 8, dans laquelle le premier anticorps conjugué est dirigé contre un déterminant antigénique de surface cellulaire différent du déterminant antigénique de surface cellulaire contre lequel est dirigé le deuxième anticorps conjugué.

11. Composition selon l'une quelconque des revendications 7 à 10, dans laquelle chaque anticorps est spécifique d'un déterminant antigénique de cellule tumorale.

12. Composition selon la revendication 7, dans laquelle le deuxième conjugué comprend un anticorps spécifique d'un déterminant antigénique de surface cellulaire et le premier conjugué comprend un anticorps spécifique de l'anticorps du deuxième conjugué.

13. Composition selon l'une quelconque des revendications 7 à 12, dans laquelle le chaîne A d'une toxine est choisie parmi le fragment chaîne A de la ricine, de l'abrine, de la modeccine, de la gélonine, de la lectine "pokeweed mitogen", de la viscumine, de la toxine du choléra, de la toxine thermolabile de *E. coli*, de la toxine de la coqueluche, de la toxine botulique, de la toxine Pseudomonas, de la toxine Shigella et de la toxine de la diphtérie.

14. Composition selon la revendication 13, dans laquelle le fragment chaîne A d'une toxine est la chaîne A de la ricine et le fragment chaîne B d'une toxine est la chaîne B de la ricine.

15. Formulation pharmaceutique qui comprend un conjugué chaîne B selon l'une quelconque des revendications 1 à 6, associé avec un ou plusieurs excipients ou véhicules pharmaceutiquement acceptables.

16. Produit contenant un conjugué de chaîne A selon l'une quelconque des revendications 7 à 14 et un conjugué de chaîne B selon l'une quelconque des revendications 1 à 6, en tant que préparation combinée pour utilisation simultanée, distincte ou successive en thérapie.

17. Conjugué de chaîne A selon l'une quelconque des revendications 7 à 14, combiné d'une manière simultanée, distincte ou successive, à un conjugué de chaîne B selon l'une quelconque des revendications 1 à 6, pour utilisation en thérapie.

18. Procédé pour la préparation d'un conjugué de chaîne B de toxine selon l'une quelconque des revendications 1 à 6, qui consiste à coupler par liaison covalente un fragment chaîne B d'une toxine à un anticorps.

## Revendications pour l'état Contractant: AT

1. Procédé pour la préparation d'un conjugué de chaîne B d'une toxine, qui consiste à coupler par liaison covalente un anticorps à un fragment chaîne B d'une toxine.

2. Procédé pour la préparation d'un conjugué selon la revendication 1, dans lequel l'anticorps est spécifique d'un antigène de surface cellulaire.

3. Procédé pour la préparation d'un conjugué selon la revendication 1 ou 2, dans lequel l'anticorps est dirigé contre un antigène de surface cellulaire d'une cellule tumorale.

4. Procédé pour la préparation d'un conjugué selon la revendication 1, dans lequel l'anticorps est dirigé contre un deuxième anticorps.

5. Procédé pour la préparation d'un conjugué selon l'une quelconque des revendications 1 à 4, dans lequel la chaîne B d'une toxine est choisie parmi la chaîne B de la ricine, la chaîne B de la modeccine, la chaîne B de l'abrine, la chaîne B de la lectine "pokeweed mitogen", la chaîne B de la viscumine, la chaîne B de la toxine du choléra, la chaîne B de la toxine thermolabile de *E. coli*, la chaîne B de la toxine de la coqueluche, la chaîne B de la toxine botulique, la chaîne de la toxine de Pseudomonas, la chaîne B de la toxine de Shigella ou la chaîne B de la toxine de la diphtérie.

6. Procédé pour la préparation d'un conjugué selon la revendication 5, dans lequel le chaîne B d'une toxine est la chaîne B de la ricine.

7. Procédé pour la préparation d'une composition cytotoxique, qui consiste à associer un premier conjugué selon l'une quelconque des revendications 1 à 6 à un deuxième conjugué comprenant un anticorps couplé par liaison covalente à un fragment chaîne A d'une toxine.

8. Procédé pour la préparation d'une composition selon la revendication 7, dans laquelle le deuxième anticorps conjugué est dirigé contre un déterminant antigénique de surface cellulaire.

9. Procédé pour la préparation d'une composition selon les revendications 7 ou 8, dans laquelle chacun du premier et du deuxième conjugués comprend un anticorps ayant une spécificité identique à un déterminant antigénique de surface cellulaire.

10. Procédé pour la préparation d'une composition selon la revendication 7 ou 8, dans laquelle le premier anticorps conjugué est dirigé contre un déterminant antigénique de surface cellulaire différent du déterminant antigénique de surface cellulaire contre lequel est dirigé le deuxième anticorps conjugué.

11. Procédé pour la préparation d'une composition selon l'une quelconque des revendications 7 à 10, dans laquelle chaque anticorps est spécifique d'un déterminant antigénique de cellule tumorale.

12. Procédé pour la préparation d'une composition selon la revendication 7, dans laquelle le deuxième conjugué comprend un anticorps spécifique d'un déterminant antigénique de surface cellulaire et le premier conjugué comprend un anticorps spécifique de l'anticorps du deuxième conjugué.

13. Procédé pour la préparation d'une composition selon l'une quelconque des revendications 7 à 12, dans laquelle la chaîne A d'une toxine est choisie parmi le fragment chaîne A de la ricine, de l'abrine, de la modeccine, de la gélonine, de la lectine "pokeweed mitogen", de la viscumine, de la toxine du choléra, de la toxine thermolabile de *E. coli*, de la toxine de la coqueluche, de la toxine botulique, de la toxine Pseudomonas, de la toxine Shigella et de la toxine de la diphtérie.

14. Procédé pour la préparation d'une composition selon la revendication 13, dans laquelle le fragment chaîne A d'une toxine est la chaîne A de la ricine et le fragment chaîne B d'une toxine est la chaîne B de la ricine.

15. Conjugué de chaîne B d'une toxine, quand il est préparé par un procédé selon l'une quelconque des revendications 1 à 6.